# EUROPEAN PATENT SPECIFICATION

(11) **EP 2 937 333 B1**
(45) Date of publication and mention of the grant of the patent: **31.08.2016**
(21) Application number: 15164708.8
(22) Date of filing: 22.04.2015
(51) Int. Cl.: C07C 231/02, C07C 233/18

(54) **PROCESS FOR PREPARING A SOLID HYDROXYALKYLAMIDE**
VERFAHREN ZUR HERSTELLUNG EINES FESTEN HYDROXYALKYLAMIDS
PROCÉDÉ DE PRÉPARATION D'UN SOLIDE À BASE D'HYDROXYALKYLAMIDE

(30) Priority: 22.04.2014 EP 14382144
(43) Date of publication of application: 28.10.2015
(73) Proprietor: Cromogenia Units, S.A., 08040 Barcelona (ES)
(72) Inventor: Torrelles Nogués, Antonio, 08040 BARCELONA (ES); Morell García, Albert, 08040 BARCELONA (ES)
(74) Representative: Oficina Ponti, SLP

(56) References cited:
- EP-A1- 0 473 380
- EP-A2- 0 960 878
- WO-A1-96/33967
- US-A- 4 076 917

## Description

### FIELD OF INVENTION

The present invention relates to a process for preparing a solid hydroxyalkylamide which is useful as an acid polyester-based curing agent for powder coating as an alternative to TGIC (Triglycidylisocyanurate).

### BACKGROUND OF THE INVENTION

Powder coating compositions allow to avoid the use of organic solvents in liquid paints. This is advantageously important since organic solvents used in liquid coatings tend to volatilize. In order to make powder coatings triglycidylisocyanurate (TGIC) and some other chemicals like hydroxylamides with two or more hydroxyl groups are used. They are useful as a crosslinker, particularly the N,N,N',N'-tetrakis(2-hydroxyethyl)adipamide, or the bis-(N,N'-di-ethylhydroxy)adipoyl amide or bis-diethanoladipamide.

Some hydroxyalkylamides (in particular, the bis-diethanoladipamide) have been synthesized as a crosslinker by reaction of dimethylesters with alkanolamines, like diethanolamine. This kind of products are obtained by recrystallizing, usually in methanol/acetone, cooling for growing the crystals, filtering and drying (see, for example, J. Coat. Tech., 50 (643), 49-55 (1978); US-A-4,032,460; US-A-4,076,917; US-A-4,493,909; US-A-4,727,111 and Japanese Patent 56-062895).

Direct flaking is not a valid option because in a molten state after some time (from some minutes until one hour as maximum) and depending basically the temperature, the hydroxyalkylamides, like bis-diethanoladipamide, degrade to low melting point mixtures, giving liquid products even at room temperature, which makes them unsuitable for use in powder coatings, since they are thermodynamically unstable, as we have demonstrated.

The recrystallization process is typically performed by dissolving the crude hydroxyalkylamide (e.g. Bis-Diethanoladipamide) in a solvent, such as methanol/acetone, cooling the solution to grow the crystals, filtering off the product from the mother liquors, and drying the product until no residual solvent is present.

Document EP0960878 discloses the preparation of beta-hydroxyalkylamide from the corresponding alkyl ester and beta-amino alcohol in the presence of a basic catalyst. Afeter the reaction is complete an acid is added to neutralize the catalyst. The obtained hydroxyalkylamide is crystallized directly.

With prilling/flaking processes, the hydroxyalkylamide (Bis-Diethanoladipamide) must be maintained in a molten state for several hours until the operation is complete. During this period of time, some of the product in the molten state degrades to undesirable by-products; making the final hydroxyalkylamide unsuitable for their use in powder coatings.
In addition, for a large scale preparation, due to the thermodynamic characteristics, this kind of products are not stable in molten state. In fact, as disclosed in the prior art, beta-hydroxyalkylamides, like bis-diethanoladipamide, must be dissolved in an organic solvent, like methanol, or even mixtures like methanol/acetone, and proceed with the production by recrystallization with cooling, or by solvent evaporation at relatively low temperature. No engines are available for flaking big amounts of this compound in a very short time, without loosing quality for the application of this solid in powder coatings.

The present invention provides a process for preparing a solid hydroxyalkylamide, in particular, bis-diethanoladipamide, overcoming the above mentioned problems associated with the known processes in the art.

### SUMMARY OF THE INVENTION

The present invention relates to a process for preparing a solid hydroxyalkylamide comprising the steps of:
(a) reacting a dimethyl ester of a C₂-C₁₂ dicarboxylic acid with an alkanolamine in the presence of a basic catalyst at a temperature in the range between 80°C and 100°C until no ester is detected in the reaction mixture;
(b) adding only water to the reaction mixture obtained in step (a);
(c) solidifying the product obtained in step (b),
wherein said dimethylester of a C2 -C12 dicarboxylic acid is at least one selected from dimethyladipate, dimethylglutarate and a mixture thereof.

In particular, the dimethyl ester of a dicarboxylic acid is dimethyladipate, the alkanolamine is diethanolamine and consequently the hydroxyalkylamide is bis-diethanoladipamide.

### DETAILED DESCRIPTION OF THE INVENTION

The present invention relates to a process for preparing a solid hydroxyalkylamide comprising the steps of:
(a) reacting a dimethyl ester of a C₂-C₁₂ dicarboxylic acid with an alkanolamine in the presence of a basic catalyst at a temperature in the range between 80°C and 100°C until no ester is detected in the reaction mixture;
(b) adding only water to the reaction mixture obtained in step (a);
(c) solidifying the product obtained in step (b),
wherein said dimethylester of a C2 -C12 dicarboxylic acid is at least one selected from dimethyladipate, dimethylglutarate and a mixture thereof.

In another embodiment, the process disclosed herein further comprises the step of:
(d) drying the solidified product obtained in step (c) only when the product needs a drying step.

In another preferred embodiment of the process disclosed herein including any of the previous embodiments, said temperature in step (a) is in the range between 80°C and 85°C.

In still another preferred embodiment of the process disclosed herein including any of the previous embodiments, the amount of water added in step (b) is between 1 and 50 wt% with respect to the amount of product (hydroxyalkylamide) obtained after step (a). It is important to emphasize that the water added in step (b) is not combined with any other substance, except for, of course, the possible impurities included in water.

This added water makes the reaction mixture stable for 8 to 10 h, avoiding at the same time the formation of undesirable products which are the cause of a less pure and soft final product, which makes the hydroxyalkylamide unsuitable for their expected use. In addition, the addition of water instead of any other solvents, makes this procedure more ecologically sustainable.

The final product obtained after step (b) may have around 1% wt of water present in said product. Nevertheless, the acceptable amount of water present in the solid product (dried or not dried) depends on the final application of the end product. If the water amount present in the final product is higher than 1%, it can be dried by using a vacuum drying system at low temperature depending on the final application of the product.

In still another preferred embodiment of the process disclosed herein including any of the previous embodiments, said basic catalyst is selected from potassium methoxide, potassium tert-butoxide and a mixture thereof.

In still another preferred embodiment of the process disclosed herein including any of the previous embodiments, the amount of catalyst is in the range from 0.1 to 1.0 wt% based on the weight of the ester of step (a).

In still another preferred embodiment of the process disclosed herein including any of the previous embodiments, the solidification step (c) is carried out by a flacking, extrusion or countercurrent spray system.

In a more preferred embodiment of the process disclosed herein including any of the previous embodiments, the dimethylester ester of a C₂-C₁₂ dicarboxylic acid is dimethyladipate, the alkanolamine is diethanolamine and the hydroxyalkylamide is bis-diethanoladipamide.

For this preferred embodiment, the reactions are shown in the following scheme:

### Scheme of reaction:

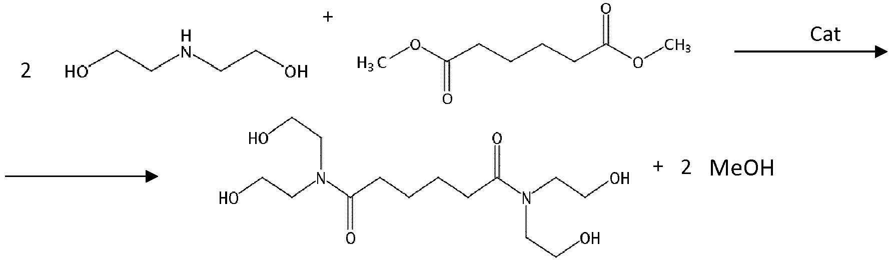

For this preferred embodiment, the undesired oligomers that could be formed are due to:
- an end product reacting with the intermediate (Dimethyladipate + Diethanolamine) 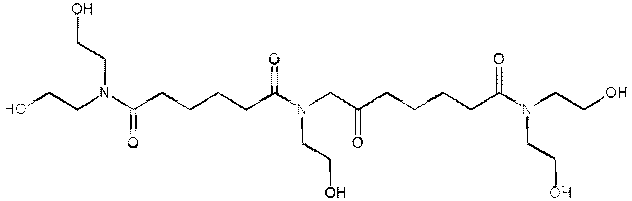
- an intramolecular reaction: 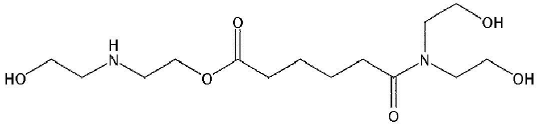
- an intermolecular reaction forming diethanolamine

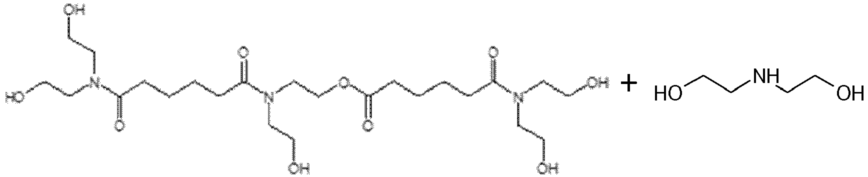

The formation of these undesirable products starts once the dimethyladipate (DMA) addition is complete. Once the ester IR band is at minimum, and the amide IR band is at maximum, if no water is added, the ester content and the amine value of the reaction mixture starts to increase reaching within few minutes values of e.g. 7.5% expressed as diethanolamine. The following table shows the amine value evolution during the reaction, caused by the diethanolamine (DEA) formation:

**Table 1**

| Minutes after DMA addition | DEA% |
|---|---|
| 0 | 9.4 |
| 10 | 7.1 |
| 15 | 4.6 |
| 20 | 2.2 |
| 30 | 3.4 |
| 40 | 5.6 |
| 60 | 7.3 |

It is important to note that the temperature of the crude reaction mixture is controlled until the hydroxyalkylamide (preferably, Bis-Diethanoladipamide) becomes a white coloured wax. It is recommendable to keep the product under stirring, but without melting the entire product. The range of temperatures in the reaction mixture is maintained between 80°C and 100°C, preferably between 80-85°C in order to maintain the product in a pasty form, without reaching the liquid state. At the same time, the alcohol obtained as by-product is distilled.

Once the addition of reactants of step (a) is complete, samples are taken continuously and the ester band is analyzed by any suitable technique, for example, infrared spectroscopy (IR) until no ester is detected. When no ester is detected, by e.g. following the IR absorption at 1700 cm⁻¹, the reaction is totally completed. This control of the reaction by following the dimethyladipate transformation provides the detection of the reaction endpoint in a more reliable way than with other control methods based on the distilled alcohol, which often is not all recovered. At this point water is added according to the conditions mentioned above.

The obtained pasty product after step (b) can be usually maintained for about 8 to 10 hours, without stability problems; no formation of by products (Diethanolamine and ester content remains constant) is observed; and finally the solidification step (c) is carried out by a suitable method including, without being limited thereto, by a flacking, extrusion or countercurrent spray system.

The optional additional drying process can be performed by a vacuum drying system with stirring and crushing. At this time, the residual alcohol obtained as a by-product, preferably methanol if the dimethyl ester of dicarboxylic acid is dimethyladipate, the alkanolamine is 2-diethanolamine and the hydroxyalkylamide is bis-diethanoladipamide, is usually removed at atmospheric or reduced pressure.

The obtained solid is a hydroxyalkylamide, preferably, bis-diethanoladipamide, having the consistency of a free-flowing powder suitable for the direct use as a crosslinker in powder coating formulations.

The solid hydroxyalkylamides, preferably, bis-diethanoladipamide, produced by the process disclosed herein have been improved compared to products produced by prior art methods of recrystallization because no solvents are involved in the final stages, no additional steps of solvent removal/recovery are necessary, and because of the improved characteristics (e.g. free-flowing powder) of the hydroxyalkylamides, preferably, bis-diethanoladipamide, which are at least equal or exceed those obtained from recrystallization.

In addition, the hydroxyalkylamide, preferably, bis-diethanoladipamide, obtained with the process disclosed herein is a product of much higher overall quality (i.e. fewer impurities are present) because said process does not subject the hydroxyalkylamides, preferably, bis-diethanoladipamide, to long hold times at elevated temperatures in the molten state during which times undesirable by-products are formed. Also, the operation of filtration, usually associated with recrystallization routes, is not necessary in the process disclosed herein.

The present invention will now be described by way of examples.

### EXAMPLES

### EXAMPLE 1 (process with no addition of water)

Diethanolamine was loaded into a glass reactor and dried under vacuum over 110°C until the water content was below 0.1 %. Once the diethanolamine was dried, and under constant nitrogen sparging, it was heated to 80-85°C and a first addition of potassium methoxide was carried out, 33% of the total amount. After the incorporation of one third of the total amount of the catalyst, the addition of dimethyladipate started and it took about 2 hours. One third of the total amount of the catalyst was added after 40 minutes and after 80 minutes since the dimethyladipate addition started .The catalyst was therefore added in 3 additions. During the addition of dimethyladipate, methanol was distilled. Once the dimethyladipate was completely added, a control of the amine value by HCIO4 titration, and the amide/ester ratio present in the reaction mixture was monitored by Infrared spectroscopy (IR) until the ester band becomes minimum and the amide band becomes maximum.

Table 1 above and table 2 below describe the increasing amine value and the decreasing melting point of the final product formed, caused by the undesirable products formation, which makes the final product dark, soft, with low melting point and not suitable for its main application.

When no water is added to the reaction mixture, as described above, the final hydroxyalkilamide formed, is not stable, and degrades to undesirable products, which makes liquid the final hydroxyalkilamide formed.

**TABLE 2 Decreasing melting point and appearance of the final product**

| **M.P. (C**°) | | **Physical Appearance** |
|---|---|---|
| **124°C** | | **White Solid** |
| 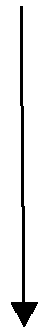 | | **White pasty liquid** |
| | | **Whitish liquid** |
| | | **Colorless liquid** |
| **25°C** | | **Dark liquid** |

### EXAMPLE 2 (Process according to the present invention)

Example 1 was repeated by following the same procedure, until finish the dimethyladipate addition. After that, the amine value by titration, and the ester content by infrared spectroscopy, was controlled constantly. Once the ester amount becomes minimum and the amide maximum; 40% of water (based on the total product amount formed) was quickly added to stop the reaction and stabilize it for 8 to 10 hours the synthetized hydroxyalkilamide.

It was very important to control the reaction continuously using infrared spectroscopy (IR), because within few minutes the product could degrade again giving esters, rising the amine value and lowering the melting point. Therefore, IR monitoring was required to follow how the reaction was taking place.

Once the water addition was completed, the transparent and liquid solution formed, was dried by using a lab scale rotaevaporator, at 65°C, and -0.8 bar. The product obtained is a white solid hydroxialkylamide with a water content less of 0.8%, a melting point of 124.7°C and an hydroxyl value of 691 mg KOH/g.

### EXAMPLE 3 (pilot plant preparation)

First it was checked that the reactor was clean and dry, and that the raw materials (dimethyladipate and diethanolamine) did not contain any moisture by Karl Fisher method. The humidity had to be less than 0.1 % wt. Diethanolamine had to be dried under vacuum in the presence of N₂ at 120-130°C to completely eliminate the moisture, until the value by the Karl Fisher method was <0.1 %. The water and acetic acid were also prepared. The preparation of 250 kg of dietanolamineadipate for a pilot plant was as follows:
Once the diethanolamine (DEA) was dried, the dried 156.5 kg of the diethanolamine in the reactor were heated between 80 and 85°C. Then 1.08 kg of catalyst (potassium methoxide) was charged, and the addition of 129.7 kg of dimethyladipate (DMAd) started and took about 2 hours. 1.08 kg catalyst was added after 40 minutes and after 80 minutes since the dimethyladipate addition started. The whole process had to be carried out under constant nitrogen sparging. During the reaction, methanol was distilled (approximately 47.7 kg), and had to be remove to keep warm the pipe to the condenser, so that methanol did not return to the reaction mixture. As the reaction took place the product became a white coloured wax. It was important to keep the product under stirring, but without melting the entire product. If necessary, the temperature was raised from 85°C to 100°C, in order to maintain the product pasty, not in liquid form. Once the addition was completed, samples were continuously taken and the ester band was analyzed by infrared spectroscopy (IR) until it disappeared or became minimum, and the amide band became maximum. At this point water is added, leaving the product with a moisture content of 3%. After the reaction for up to 7 to 8 hours the product is maintained stable, and it could be flaked for solidification and preserved.

Experimental conditions and analytical data obtained from on-line sampling are presented in Table 3.

**TABLE 3**

| Time (min) | Temperature (°C) | Operation |
|---|---|---|
| 0 | | DEA charged and add 1/3 catalyst and start adding DMAd |
| 49.00 | 84.20 | Add 1/3 catalyst |
| 80.00 | 84.60 | Distillation begins very slowly |
| 87.00 | 87.30 | The temperature is raised to 88-90°C |
| 103.00 | 88.90 | Add 1/3 catalyst |
| 158.00 | 88.40 | Addition of DMAd is complete |
| 193.00 | 85.10 | Pasty white product |
| 210.00 | 88.10 | Adding water |

| | | |
|---|---|---|
| DEA: 2-diethanolamine DMAd: dimethyladipate | | |

**TABLE 2**

| Physical Appearance of final product | White solid |
|---|---|
| % residual DEA (titration with HClO4) | 2% |
| M.P. (°C) (DSC) | 124°C |

## Claims

1. Process for preparing a solid hydroxyalkylamide comprising the steps of:
(a) reacting a dimethylester of a C₂-C₁₂ dicarboxylic acid with an alkanolamine in the presence of a basic catalyst at a temperature in the range between 80°C and 100°C until no ester is detected in the reaction mixture;
(b) adding only water to the reaction mixture obtained in step (a);
(c) solidifying the product obtained in step (b);
wherein said dimethylester of a C₂-C₁₂ dicarboxylic acid is at least one selected from dimethyladipate, dimethylglutarate and a mixture thereof.

2. Process according to claim 1, further comprising the step of:
(d) drying the solidified product obtained in step (c).

3. Process according to any of the preceding claims, wherein said temperature is in the range between 80°C and 85°C.

4. Process according to any of the preceding claims, wherein the amount of water added in step (b) is between 1 and 50 wt% with respect to the amount of product obtained after step (a).

5. Process according to any of the preceding claims, wherein said basic catalyst is selected from potassium methoxide, potassium tert-butoxide and a mixture thereof.

6. Process according to any of the preceding claims, wherein the amount of catalyst is in the range from 0.1 to 1.0 wt% based on the weight of the ester of step (a).

7. Process according to any of the preceding claims, wherein the solidification step (c) is carried out by a method selected from flaking, extrusion and countercurrent-spray system.

8. Process according to any of the preceding claims, wherein the alkanolamine is selected from diethanolamine, monoethanolamine and diisopropanolamine.

9. Process according to any of the preceding claims, wherein the dimethylester of a C₂-C₁₂ dicarboxylic acid is dimethyladipate, the alkanolamine is 2-diethanolamine and the hydroxyalkylamide is bis-diethanoladipamide.

## Patentansprüche

1. Prozess zum Präparieren eines festen Hydroxyalkylamids, das folgende Schritte aufweist:
(a) Reagierenlassen eines Dimethylesters einer C₂-C₁₂-Dicarbonsäure mit einem Alkanolamin in Anwesenheit eines basischen Katalysators bei einer Temperatur in dem Bereich zwischen 80°C und 100°C, bis kein Ester in dem Reaktionsgemisch erfasst wird;
(b) Hinzufügen von ausschließlich Wasser zu dem in Schritt (a) erhaltenen Reaktionsgemisch;
(c) Verfestigen des in Schritt (b) erhaltenen Produkts;
wobei der Dimethylester einer C₂-C₁₂-Dicarbonsäure zumindest einer ausgewählt aus Dimethyladipat, Dimethylglutarat und einem Gemisch derselben ist.

2. Verfahren gemäß Anspruch 1, das ferner folgenden Schritt aufweist:
(d) Trocknen des in Schritt (c) erhaltenen verfestigten Produkts.

3. Verfahren gemäß einem der vorherigen Ansprüche, bei dem die Temperatur in dem Bereich zwischen 80°C und 85°C liegt.

4. Verfahren gemäß einem der vorherigen Ansprüche, bei dem die Wassermenge, die in Schritt (b) hinzugefügt wird, zwischen 1 und 50 Gew.-% bezüglich der Menge des nach Schritt (a) erhaltenen Produkts liegt.

5. Verfahren gemäß einem der vorherigen Ansprüche, bei dem der basische Katalysator aus Kaliummethoxid, Kalium-tert-Butoxid und einem Gemisch derselben ausgewählt ist.

6. Verfahren gemäß einem der vorherigen Ansprüche, bei dem die Katalysatormenge in dem Bereich von 0,1 bis 1,0 Gew.% basierend auf dem Gewicht des Esters aus Schritt (a) liegt.

7. Prozess gemäß einem der vorherigen Ansprüche, bei dem der Verfestigungsschritt (c) mittels eines Verfahrens ausgewählt aus Flockung, Extrudieren und einem Gegenstrom-Sprühystem ausgeführt wird.

8. Verfahren gemäß einem der vorherigen Ansprüche, bei dem das Alkanolamin aus Diethanolamin, Monoethanolamin und Diisopropanolamin ausgewählt ist.

9. Verfahren gemäß einem der vorherigen Ansprüche, bei dem der Dimethylester einer C₂-C₁₂-Dicarbonsäure Dimethyladipat ist, das Alkanolamin 2-Diethanolamin ist und das Hydroxyalkylamid bis-Diethanoladipamid ist.

## Revendications

1. Procédé de préparation d'un hydroxyalkylamide solide comprenant les étapes suivantes :
(a) faire réagir un diméthylester d'un acide dicarbolyxique C₂-C₁₂ avec une alkanolamine en présence d'un catalyseur basique à une température comprise entre 80°C et 100°C jusqu'à ce que plus aucun ester ne soit détecté dans le mélange réactionnel ;
(b) n'ajouter que de l'eau au mélange réactionnel obtenu à l'étape (a) ;
(c) solidifier le produit obtenu à l'étape (b) ; dans lequel ledit diméthylester d'acide dicarboxylique C₂-C₁₂ est au moins l'un de ceux sélectionnés parmi le diméthyladipate, le diméthylglutarate et un mélange de ceux-ci.

2. Procédé selon la revendication 1 comprenant en outre l'étape suivante :
(d) sécher le produit solidifié obtenu à l'étape (c).

3. Procédé selon une quelconque des revendications précédentes, dans lequel ladite température est comprise entre 80°C et 85°C.

4. Procédé selon une quelconque des revendications précédentes, dans lequel la quantité d'eau ajoutée à l'étape (b) est comprise entre 1 et 50 wt% par rapport à la quantité de produit obtenu après l'étape (a).

5. Procédé selon une quelconque des revendications précédentes, dans lequel ledit catalyseur basique est sélectionné parmi du méthanolate de potassium, du tert-Butylate de potassium et un mélange de ceux-ci.

6. Procédé selon une quelconque des revendications précédentes, dans lequel la quantité de catalyseur est comprise entre 0,1 et 1,0 wt% en se basant sur le poids de l'ester de l'étape (a).

7. Procédé selon une quelconque des revendications précédentes, dans lequel l'étape de solidification (c) est mise en oeuvre selon une méthode sélectionnée parmi la floconisation, l'extrusion et un système de pulvérisation à contre-courant.

8. Procédé selon une quelconque des revendications précédentes, dans lequel l'alkanolamine est sélectionnée parmi de la diéthanolamine, de la monoéthanolamine et de la diisopropanolamine.

9. Procédé selon une quelconque des revendications précédentes, dans lequel le diméthylester d'acide dicarboxylique C₂-C₁₂ est du diméthyladipate, l'alkanolamine est du 2-diéthanolamine et l'hydroxyalkylamide est du bis-diéthanoladipamide.
